Europäisches Patentamt

European Patent Office

Office européen des brevets

(11) Veröffentlichungsnummer: **0 183 097**
**B1**

(12) **EUROPÄISCHE PATENTSCHRIFT**

(45) Veröffentlichungstag der Patentschrifft:
21.03.90

(51) Int. Cl.⁵: **G 01 N   1/10, B 01 L   3/00**

(21) Anmeldenummer: **85114092.1**

(22) Anmeldetag: **05.11.85**

(54) Verfahren zur Probennahme und Zuordnung zwischen Probenmengenlieferant und Probengefäss.

(30) Priorität: **07.11.84 DE 3440686**

(43) Veröffentlichungstag der Anmeldung:
**04.06.86 Patentblatt 86/23**

(45) Bekanntmachung des Hinweises auf die Patenterteilung:
**21.03.90 Patentblatt 90/12**

(54) Bennante Vertragsstaaten:
**AT BE CH DE FR GB LI NL**

(56) Entgegenhaltungen:
**EP-A-0 015 531
DE-A-2 753 865
DE-A-2 913 989**

(73) Patentinhaber: **OTTO TUCHENHAGEN GmbH & Co. KG
Berliner Strasse 10 Postfach 1140
D-2059 Büchen (DE)**

(72) Erfinder: **Mieth, Hans Otto
Sandkrug 3
D-2058 Schnakenbek/Lbg (DE)**

(74) Vertreter: **Glaeser, Joachim, Dipl.-Ing.
Patentanwälte DIEHL GLAESER HILTL & PARTNER
Königstrasse 28
D-8000 Hamburg 50 (DE)**

**Beschreibung**

Die Erfindung bezieht sich auf ein Verfahren zur Probennahme und Zuordnung zwischen Probemengenlieferant und Probengefäß, insbesondere bei der Milchübernahme aus Behältern verschiedener Lieferanten (identisch mit Probemengenlieferant) in einen Sammelbehälter, der mit seinem Annahme- und Meßsystem während der Überführung der Gesamtmenge in einen Sammelbehälter eine Probenmenge entnimmt und diese in ein Probengefäß füllt.

Bei der Probennahme von Milch über das Annahmesystem des Milchsammelwagens (Sammelbehälter) wird die aus einem sogenannten Probenvorlaufgefäß abgeteilte Probenmenge in ein Probengefäß überführt. Dabei besteht das Problem, die genommene Milchprobe eindeutig zu definieren, das heißt festzuhalten, von welchem Probemengenlieferanten die Milchprobe erfolgt, da nachfolgend zumeist ein Transport der Milchprobe vom Ort der Probennahme zum Labor, das diese analysiert, erforderlich ist.

Das Problem wird noch weiter erschwert, wenn ein Milchsammelwagen – dies ist sein eigentlicher Zweck – eine Vielzahl von Milcherzeugern, von denen jeder auch ein Probemengenlieferant sein kann, nacheinander anfährt, um vor Ort Probenmengen in die Probengefäße abzufüllen. Dabei ist es nicht außergewöhnlich, wenn auf einer sogenannten Milcherfassungstour weit mehr als hundert Milcherzeuger (im folgenden Lieferant oder Probemengenlieferant genannt) in im vorhinein bestimmter oder aber auch von Zufälligkeiten oder Irrtümern seitens der Milchsammelwagenfahrer abhängiger Reihenfolge nacheinander aufgesucht werden.

Es ist mehr oder weniger selbstverständlich, zur Aufbewahrung und zum Transport der Milchprobe eine Flasche zu verwenden, die dann selbst oder deren Aufbewahrungsort zum Zwecke der Zuordnung zwischen Probemengenlieferant und Probengefäß eine Kennzeichnung erhält. Es wird also nicht die Probe selbst, sondern das Probengefäß einem bestimmten Probemengenlieferanten an Ort und Stelle zugeordnet.

Im Zusammenhang mit diesem bekannten Verfahren können eine Reihe von Fehlern auftreten. Diese Fehler lassen sich jedoch auch noch nicht dadurch ausschließen, daß in Vorbereitung einer Probennahme die Probengefäße eine Kennzeichnung erhalten, und die so gekennzeichneten Probengefäße bei der Probennahme dem Probemengenlieferanten zugeordnet werden. Bei dieser Vorgehensweise sind eine Reihe von Fehlern nicht auszuschließen.

So kann beispielsweise die Kennzeichnung bzw. die Kodierung fehlerhaft, unleserlich oder beschädigt sein, so daß eine Zuordnung des Probengefäßes zum jeweiligen Lieferanten nicht möglich ist.

Ein Verfahren, das die vorstehenden Nachteile aufweist, ist aus der DE-A-2 753 865 bekannt. Es sieht vor, daß aus wenigstens einem Folienmaterial in einer kettenartigen Anordnung aneinandergereihte Behälter für die Milchproben gebildet werden, daß jeder Behälter mit einer die darin enthaltene Milchprobe betreffenden Kennzeichnung versehen wird und daß die Behälter jeweils nach dem Befüllen mit einer Milchprobe verschlossen werden. Darüber hinaus stellt die zur Kennzeichnung der Behälter am Ort der Probenahme, also auf dem Milchsammelwagen, erforderliche Markierungsstation eine weitere Störungsquelle dar, die die bei derartigen Probenahmesystemen zu fordernde Sicherheit in Frage stellt.

Ähnliche Nachteile, wie sie sich vorstehend bei der Kennzeichnung der aus Folienbändern gebildeten Behälter einstellen, ergeben sich auch bei der Handhabung einer aus der DE-A-2 758 437 bekannten Probeflasche, bei der im Bereich des Umfanges der Flasche ein Magnetspeicher angeordnet ist, der in einer Analysiereinrichtung in einer vorgebbaren Stellung der Flasche auszulesen ist. Der Beschreibung des bekannten Gegenstandes ist zu entnehmen, daß die wesentlichen Kenndaten einer Milchprobe unlösbar mit der Milchprobenflasche selbst verbunden sind, so daß keine Gefahr besteht, daß die eindeutige Zuordnung dieser Kenndaten zu dem Inhalt der Milchprobenflasche verloren gehen könnte.

Dieses bedeutet jedoch, daß die Kenndaten einer Milchprobennahme, die ja nur vor Ort gewonnen werden können, auch nur vor Ort, d. h. also auf dem Milchsammelwagen selbst, dem Probengefäß zugeordnet werden können.

Da die Probengefäße bei der Identifizierung an einem Lesegerät vorbeigeführt und anschließend in eine Position unterhalb des Probenabfüllventils gebracht werden müssen, ist apparativ sicherzustellen, daß fehlerhafte Probengefäße auf diesem Wege aussortierbar und Manipulationen an oder mit diesen Probengefäßen unmöglich sind. Es zeigt sich, daß ein derartiges Transportsystem für Probengefäße nicht unproblematisch ist. Ein weiterer und schwerwiegender Nachteil ist dadurch gegeben, daß das Kodier- und/oder Lesegerät, eine feinmechanisch-elektronische Apparatur, den rauhen Betriebsbedingungen unterworfen wird, wie sie beispielsweise auf einer Milcherfassungstour auftreten. Temperaturschwankungen, Erschütterungen, Staub, Schmutz und Feuchtigkeit beeinträchtigen deren Funktion und Betriebssicherheit. Abgesehen davon bedingt ein derartiges Verfahren auch eine voluminöse Vorrichtung für die Probengefäßumladung und Probengefäßkodierung bzw. für das Lesen der kodierten Probengefäße. Dieser Nachteil fällt besonders ins Gewicht, da das zur Unterbringung des Annahme- und Meßsystems vorhandene Raumangebot am Milchsammelwagen stets begrenzt ist und das zur Probengefäßkodierung und Probengefäßerkennung notwendige Mehrvolumen zu Lasten des Volumens des Sammelbehälters geht oder aber zu einer Vergrößerung des Fahrgestells des Milchsammelwagens führt.

Bei einem anderen bekannten Verfahren werden die Probengefäße in einem Stativ untergebracht, wobei die einzelnen Plätze oder Positionen der Probengefäße gekennzeichnet sind. Soll eine Probe genommen werden, so muß nunmehr zunächst mit Hilfe eines geeigneten Gerätes eine Kennung, ein Kennzeichen oder der Name des Probemengenlieferanten erfaßt werden. So wird beispielsweise an einem die anzunehmende Milch aufnehmenden Anlieferungsbehälter mit Hilfe eines Lesegerätes eine Lieferantenkennung gelesen. Nunmehr kann diese Lieferantenkennung einem bestimmten Platz zugeordnet werden, nämlich demjenigen, bei dem sich das Probengefäß befindet, in das die entsprechende Probe gefüllt wurde. Solange wie die Probengefäße an ihren vorgesehenen Stellen verbleiben, besteht eine Zuordnung zwischen der Kennung des Lieferanten und dem entsprechenden Platz des Probengefäßes. Verbleiben die einzelnen Probenbehälter an Ort und Stelle bis sie in das Labor gelangen, so können dort die erforderlichen Messungen durchgeführt und die Meßergebnisse der Probe dem jeweiligen Lieferanten zugeordnet werden. Wenn allerdings bei der Ablieferung der einzelnen Probengefäße – es können sich, wie einleitend erwähnt, weit mehr als hundert Probengefäße in einem aus mehreren Stativen gebildeten Magazin befinden – diese versehentlich aus dem Stativ herausfallen oder wenn beim Umladen der Probengefäße in das Analysengerät deren Reihenfolge vertauscht wird, so ist keine Zuordnung mehr möglich, nämlich welche Probengefäße zu welchem Lieferanten gehören. ·

Aber selbst im Normalbetrieb und ohne Störungen ist für spätere Reklamationen keine Möglichkeit gegeben, etwa bestimmte Messungen zu wiederholen oder dem Probemengenlieferanten die Probe zu Messungen an anderer Stelle zur Verfügung zu stellen, es sei denn, jedes Probengefäß würde in einem zusätzlichen Arbeitsgang mit einer von der Position im Stativ unabhängigen Kennung versehen, wobei eine Zuordnung zum jeweiligen Lieferanten erfolgen müßte. Diese Zuordnung wäre in irgendeiner Form datenmäßig zu erfassen und zu speichern. Um das vorgenannte Verfahren absolut sicher zu machen, sind offensichtlich eine Reihe von wirtschaftlich nicht vertretbaren Maßnahmen notwendig.

Die Analysepraxis zeigt darüber hinaus, daß in der Regel ein Verbleiben der Probengefäße im Stativ im Zuge der Analyse nicht möglich ist, da die in den Milcherzeugerregionen bzw. in den verschiedenen Milchsammelwagenfabrikaten verwendeten Stative und Magazine nicht standardisiert sind und daher auch von den Analysegeräten nicht immer angenommen werden können. Die Probengefäße müssen daher oft aus dem Stativ gelöst und in das automatisch arbeitende · Analysengerät umgeladen werden.

Aus der DE-OS-2 913 996 ist eine Einrichtung bekannt, bei der die Probennahmevorrichtung im Milchsammelwagen eine Probengefäß-Kennzeichnungsvorrichtung enthält, welche gleichzeitig mit oder abhängig von einer Auswahlvorrichtung gesteuert ist. Dadurch ist zwar eine eindeutige Zuordnung zwischen dem Probemengenlieferanten und dem Probengefäß sichergestellt, jedoch erfolgt diese Zuordnung an Ort und Stelle der Probennahme im Milchsammelwagen und unter Zuhilfenahme einer Einrichtung, die in jedem Probennahmesystem, also in jedem Sammelwagen und mit den vorstehend beschriebenen Nachteilen, bereitzustellen ist. Ohne diese Markierungsvorrichtung wäre eine eindeutige und unverwechselbare Zuordnung zwischen Probemengenlieferant und Probengefäß nicht sichergestellt, da ansonsten die Kopplung zwischen Probemengenlieferant und Probengefäß nur über die Position des Probengefäßes im Stativ gegeben und auch nur solange sichergestellt ist, wie das Probengefäß an seinem Platz zum Zeitpunkt der Probenüberführung verbleibt.

Im übrigen ist als weiterer Nachteil erkennbar, daß der Kennzeichnungsvorrat der vorgeschlagenen Markierungsvorrichtung begrenzt ist, und somit zumindest theoretisch gleichartig gekennzeichnete Probengefäße verschiedener Stative oder Magazine im Analysenlabor vorliegen können. Beim Umladen dieser Probengefäße in das Analysengerät sind diese dann nicht mehr eindeutig einem bestimmten Lieferanten zuzuordnen.

Die Erfindung befaßt sich mit der Lösung der oben angegebenen Probleme, und es soll insbesondere ein Verfahren geschaffen werden, das Fehlermöglichkeiten bekannter Verfahren vermeidet. Darüber hinaus soll erreicht werden, daß die in einem bestimmten Probengefäß ($F_i$) befindliche Probe von der Probennahme ab eindeutig einem bestimmten Lieferanten ($L_k$) zugeordnet bleibt, und zwar selbst dann, wenn die Probengefäße ($F_i$) in einer Reihenfolge geprüft werden, die nicht mit der Reihenfolge der Probennahme selbst übereinstimmt. Erreicht wird dieses durch ein Verfahren nach den kennzeichnenden Merkmalen der Patentansprüche.

Im Zusammenhang mit der Erfindung wird von einer Kennung eines Lieferanten ($L_k$) gesprochen, und hierunter soll ein Name, eine Kode-Nummer oder eine Abkürzung oder dergleichen verstanden werden. Im Zusammenhang mit Probengefäßen ($F_i$) wird von einer Kodierung gesprochen, die sich an dem Probengefäß befindet. Hier kommen alle möglichen bekannten Kodes in Frage, die beispielsweise mit maschinellen Geräten gelesen werden könne. Im Zusammenhang mit der Anordnung der Probengefäße in einem Stativ oder dergleichen wird von einer Position ($P(X_i; Y_i)$) gesprochen. Beispielsweise können die Probenbehälter mit der Angabe ihrer Orthogonalkoordinaten festgelegt werden. Eine Festlegung über andere bekannte Koordinatensysteme ist ebenfalls möglich.

Wesentlich für die vorliegende Erfindung ist, daß die Probengefäße ($F_i$) mit einer Kodierung versehen werden. Diese Kodierung wird gemäß der Erfindung nicht an Ort und Stelle der Probennahme, sondern unter den einwandfreien Bedingungen eines Betriebes, z. B. einer Molkerei oder eines Zentrallabors oder dergleichen durchgeführt.

Durch die erfindungsgemäß vorgeschlagene Kodierung der Probengefäße in Verbindung mit deren Handhabung und Zuordnung zu den jeweiligen Probemengenlieferanten wird es erstmals möglich, die unterschiedlichen Probengefäß-Stative und -Magazine der verschiedenen am Markt befindlichen Milchsammelwagen, deren unterschiedliche Ausführungen nicht nur bauartbedingt, sondern auch regional bedingt sind, in das meist zentralistisch organisierte Analysengeschehen einzubeziehen, ohne daß eine Umrüstung der in der Regel nicht analysengerätekompatiblen Stative oder Magazine auf die installierten Analysengeräte bzw. Anpassung der Analysengeräte an die vorliegenden Stativ- oder Magazinsysteme erforderlich ist. Obgleich das vorgeschlagene Verfahren ein Maximum an Flexibilität im vorstehenden Sinne ermöglicht, ist eine nicht mehr erhöhbare Sicherheit in Bezug auf die Zuordnung und Unverwechselbarkeit zwischen Lieferant ($L_k$) und Probengefäß ($F_l$) gewährleistet.

Beispielsweise können Probengefäße verwendet werden, die mit einem Etikett, einem sogenannten Barcode, versehen sind. Vor der Anordnung der Probenbehälter an bestimmter Stelle wird diese Kennzeichnung mit Hilfe eines maschinellen Gerätes gelesen. Gemäß der Erfindung können mehrere derartige Lesevorgänge durchgeführt werden, um sicherzustellen, daß der Kode eindeutig zu erkennen ist. Erweist sich in diesem Zusammenhang eine Kodierung als unsicher oder nicht lesbar, so wird das entsprechende Probengefäß aussortiert und/oder mit einer einwandfreien Kodierung versehen. Bei dem Verfahren gemäß der Erfindung ist kurz vor der Einordnung in das Stativ entweder das Versehen der Probengefäße mit Kodierungen und das Lesen dieser Kodierungen oder aber das einwandfreie Herauslesen bereits zuvor kodierter Probengefäße erforderlich.

Die einwandfrei identifizierbaren Probenbehälter werden in einem Stativ oder dergleichen angeordnet, wobei die Probenbehälter im Stativ so befestigt werden können, daß sie nicht mehr aus dem Stativ entnommen werden können. Beispielsweise können die Behälter in ihrer Gesamtheit an der vorgesehenen Stelle plombiert werden, wobei die Plombierung erst nach Rücklieferung der Milchproben geöffnet wird. Dadurch werden mögliche Manipulationen ausgeschlossen.

In jedem Falle werden die einzelnen Positionen ($P(X_i; Y_j)$) zusammen mit den Kodierungen der Probenbehälter ($F_l$) gespeichert, so daß von diesem Zeitpunkt an bekannt ist, welcher spezielle Probenbehälter sich an welcher Position befindet. Selbstverständlich können auch die Stative oder Probenbehältereinrichtungen identifiziert werden, so daß aus einem Speicher abgefragt werden kann, welche Probenbehälter sich in welchem Stativ an welcher Stelle befinden.

Nachfolgend wird von einem Stativ gesprochen, obwohl natürlich die vorliegende Erfindung nicht auf den Einsatz eines Stativs beschränkt ist. Das Stativ wird nunmehr mit den leeren Probenbehältern zu einem Probensammelgerät gebracht, beispielsweise zu einem Milchsammelwagen, der zu den einzelnen Milchlieferanten fährt, um dort vor Ort jeweils eine Probe zu nehmen.

Ein solcher Milchsammelwagen kann mit einem Datenerfassungsgerät versehen sein, welches Informationen darüber enthält, welche Probenbehälter sich an welcher Stelle im Stativ befinden ($F_l \rightarrow P(X_i; Y_j)$).

Darüber hinaus enthält dieses Gerät auch Informationen darüber, welche Lieferanten der Milchsammelwagenfahrer und in welcher Reihenfolge er diese aufsuchen soll. Hält sich der Fahrer an diese Reihenfolge, so ist gemäß der Erfindung eindeutig sichergestellt, daß sich in einem Probenbehälter mit einer bestimmten Kodierung ($F_l$) die Probe eines bestimmten Probemengenlieferanten mit einer bestimmten Kennung ($L_k$) befindet. Die Proben können aus dem Stativ entnommen und in beliebiger Reihenfolge untersucht werden.

Hält sich der Milchsammelwagenfahrer aus irgendeinem Grunde nicht an diese Reihenfolge, so kann er in sein Datenerfassungsgerät eingeben, an welcher Stelle er gerade eine Milchprobe nimmt. Er gibt dazu die Kennung des Lieferanten ($L_k$) ein, und das Datenerfassungsgerät ordnet diese Kennung dem bestimmten Probenbehälter bzw. der bestimmten Position ($P(X_i; Y_j)$) zu.

Es besteht auch die Möglichkeit, daß der Fahrer mit Hilfe des Datenerfassungsgerätes die Reihenfolge ändert, in der er die einzelnen Lieferanten anfährt, wobei er sein Datenerfassungsgerät befragt, welche Lieferanten aufgesucht werden sollen, und er durch eine entsprechende Eingabe diese Reihenfolge mit der Reihenfolge verbindet, in der die einzelnen Milchproben in die Probenbehälter abgegeben werden. In jedem Falle ist sichergestellt, daß die Zuordnung zwischen Kennung (Probemengenlieferant) und Position (Stativ) bzw. Kodierung (Probengefäß) festgehalten wird.

Die Lieferantenerkennung kann auch durch Lesen einer Kennung des Anlieferungsbehälters oder einer Lieferantenkodierung am Ort der Milchannahme erfolgen. In diesem Falle ergibt sich für das Datenerfassungsgerät immer noch die Zuordnungsaufgabe im vorgenannten Sinne.

Es besteht weiterhin die Möglichkeit, daß am Ort der Probennahme das Datenerfassungsgerät einen Beleg in zweifacher Ausfertigung ausdruckt, von dem ein Exemplar dem Lieferanten zur Verfügung gestellt werden kann. Dieser Beleg kann beispielsweise die Kodierung des Probenbehälters und die Kennung des Lieferanten enthalten, so daß dieser eine Möglichkeit hat, eine Reklamation im Zusammenhang mit den Meßwerten im Labor anzubringen. Selbstverständlich können auch noch weitere Daten auf diesem Beleg ausgedruckt werden, wie z. B. die Milchmenge.

Die Erfindung weist weiterhin noch Vorteile im Zusammenhang mit der Aufbereitung der Probengefäße auf. Bevor nämlich die Probengefäße kodiert und anschließend kontrollgelesen und an bestimmter Stelle in einem Probensammelgerät untergebracht werden, können die Probengefäße einzeln oder gemeinsam gereinigt, mit einem Hemmstoff gefüllt und verkorkt werden.

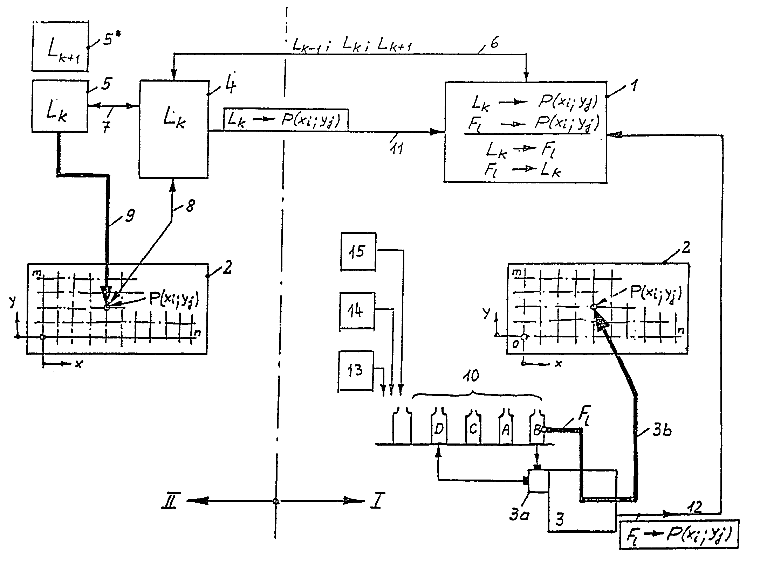

Die Erfindung wird nachstehend an Hand der Zeichnung beispielsweise erläutert.

Die Figur zeigt eine schaubildliche Darstellung zur Erläuterung des Verfahrens gemäß der Erfindung.

Im rechten Teil der Darstellung (Ort I) sind die Vorgänge dargestellt, die sich beispielsweise in einer Molkerei oder in einem Zentrallabor abspielen, während im linken Teil (Ort II) diejenigen Vorgänge gezeigt sind, die sich vor Ort, nämlich bei einem Milchlieferanten abspielen.

Mit 1 ist eine Datenerfassungszentrale bezeichnet, welche bestimmte Informationen in einer Zuordnung zueinander enthält. $L_k$ ist die Kennung eines bestimmten Lieferanten, ($P(X_i; Y_j)$) sind die Koordinaten eines bestimmten Punktes in einem Probengefäßmagazin 2 · $F_l$ ist die Kodierung eines bestimmten Probengefäßes, das sich an der vorgenannten Stelle befindet. Aus diesen Zuordnungen läßt sich eindeutig eine Kennung eines Lieferanten mit der Kodierung eines Probengefäßes erreichen.

Mit 13, 14 und 15 sind bestimmte Einrichtungen oder Verfahrensstufen bezeichnet, beispielsweise die einer Reinigungsanlage, einer Hemmstoffzuführungseinrichtung und einer Verkorkungsstelle.

Mit 3 ist eine Lese- und Kodiereinrichtung mit Ladegerät für die Probengefäße bezeichnet. An der Stelle 3a wird die auf einem Probengefäß befindliche Kodierung gelesen, und ggf. wird vorher dieses Probengefäß kodiert. Mit 10 sind beispielsweise vier Probengefäße dargestellt, die die Kodierung D, C, A und B tragen. Die Leseeinrichtung 3a liest diese Kodierung ab und gibt über die Leitung 12 die entsprechende Information zur Datenerfassungszentrale 1. Weiterhin wird dorthin die Stelle angegeben, wo das entsprechende Probengefäß in das Magazin gebracht wird.

Das Magazin wird nach der Plombierung zum Milchsammelwagen gebracht und zur Probennahmestelle gefahren. Dem Fahrer steht eine Einrichtung 4 zur Verfügung, die alle erforderlichen Informationen über die einzelnen Lieferanten enthält. Mit Hilfe dieser Einrichtung kann der Fahrer abfragen, wo er planmäßig hinfahren soll. Im dargestellten Beispiel fährt er zu dem Lieferanten $L_k$. Erreicht er diesen Lieferanten (Block 5), so nimmt er die dort bereitgestellte Milch auf und gibt diese in den Sammelbehälter.

Bei diesem Vorgang wird eine Probenmenge abgezapft und über eine Leitung 9 zu dem Probengefäß gebracht, das sich an der Position $P(X_i; Y_j)$ im Magazin befindet. Die entsprechende Information, daß nämlich die Milch dort abgeliefert worden ist, wird über die Leitung 8 in das Datenerfassungsgerät 4 eingegeben. Zur Positionsbestimmung des Probenabfüllventils (ohne Bezeichnung am Ende der Leitung 9) sieht das Verfahren drei Ausführungsvarianten vor. Die einfachste Variante geht von einer Ausgangsstellung (X = 1; Y = 1 aus und zählt in jeder Zeile ($Y_j$) die jeweiligen Schaltschritte ($X_i$). Durch eine geeignete Transporteinrichtung für das Probenabfüllventil ist jederzeit bekannt, an welcher Position $P(X_i; Y_j)$ sich das Probenabfüllventil befindet. Die Ausführungsvariante II sieht jeweils eine Rückmeldung vor, wenn das Probenabfüllventil die Endposition in jeder Zeile ($Y_j$) erreicht hat. Die Ausführungsvariante III sieht die Rückmeldung jeder einzelnen Position $P(X_i; Y_j)$ vor.

Befindet sich der Milchsammelwagenfahrer jedoch nicht beim Lieferanten $L_k$, sondern beispielsweise beim Lieferanten $L_{k+1}$ (Block 5*), so hat er die Möglichkeit, die aufgenommene Milchprobe trotzdem in das Probengefäß an der Stelle $P(X_i; Y_j)$ abzugeben, was ohne weiteres möglich ist, da diese Tatsache über die Leitung 8 in das Datenerfassungsgerät 4 eingegeben wird. Nach Rückkehr zur Molkerei bzw. zum Zentrallabor werden die entsprechenden Informationen des Datenerfassungsgerätes 4 über die Leitung 11 in die Datenerfassungszentrale 1 eingegeben. Demzufolge wird bei der Prüfung der Milchprobe an der Position $P(X_i; Y_j)$ diese Milchprobe eindeutig dem richtigen Lieferanten zugeordnet.

Alternativ zu der vorstehend vorgeschlagenen Lösung ist es selbstverständlich auch möglich, die Zuordnung zwischen Lieferant $L_k$ und Probengefäß $F_l$ in dem Datenerfassungsgerät 4 durchzuführen. Zu diesem Zweck muß vor der Milcherfassungstour dem Datenerfassungsgerät 4 die Zuordnung $F_l \rightarrow P(X_i; Y_j)$ über die Datenerfassungszentrale 1 übermittelt werden. Am Ende der Milcherfassungstour wird dann die Zuordnung $L_k \rightarrow F_l$ bzw. $F_l \rightarrow L_k$, beispielsweise über die Leitung 11, an die Datenerfassungszentrale 1 übermittelt. Ein Duplikat dieser Zuordnungsdaten begleitet die Probengefäße $F_l$ mit den Milchproben ggf. in das zentrale Analysenlabor, damit dort die Untersuchungsergebnisse über die Kodierung des Probengefäßes $F_l$ dem Lieferanten $L_k$ direkt zugeordnet werden können. Es ist auch denkbar, daß die Untersuchungsergebnisse dort lediglich dem jeweiligen Probengefäß $F_l$ zugeordnet werden, und daß die Zuordnung zum Lieferanten $L_k$ erst bei Rückgabe dieser Daten in der Molkerei erfolgt.

Abschließend werden die Lösungsmerkmale und der Ablauf des vorgeschlagenen Verfahrens noch einmal zusammengefaßt:

1. Die Probengefäße werden mit einer Kodierung ($F_l$) versehen, und die Kodierung dieser und ggf. bereits zuvor kodierter Probengefäße wird mindestens einmal gelesen (Lese- und Kodiereinrichtung 3a).
2. Danach werden die Probengefäße ($F_l$) in eine beliebige aber definierte Position ($P(X_i; Y_j)$) eines Stativs oder dergleichen verbracht (Ladegerät 3).

Vor der Kodierung und vor der Unterbringung in einem Stativ oder dergleichen lassen sich die Probengefäße, falls notwendig, aufbereiten. Sie können beispielsweise einzeln oder gemeinsam gereinigt werden, mit einem Hemmstoff gefüllt und verkorkt werden.

3. Die Zuordnung Probengefäß/Position ($F_l \rightarrow P(X_i; Y_j)$) wird gespeichert.

Dieses geschieht, und das ist der wesentliche Unterschied zu bekannten Verfahren, nicht an Ort und Stelle der Probennahme (Ort II), sondern unter den einwandfreien Bedingungen eines Betriebes, z.B. einer Molkerei oder eines Zentrallabors (Ort I).

4. Eine Milchprobe eines bestimmten Probemengenlieferanten ($L_k$) wird in ein beliebiges, an einer bestimmten Position ($P(X_i; Y_j)$) befindliches Probengefäß ($F_l$) überführt (Ort II).

5. Die Zuordnung Probemengenlieferant/Position ($L_k \rightarrow P(X_i; Y_j)$) wird gespeichert (Ort II).

6. Die Kodierung der sich an dieser Position ($P(X_i; Y_j)$) befindlichen Probenflasche ($F_l$) wird über diese Position ($P(X_i; Y_j)$) der Kennung des bestimmten Lieferanten ($L_k$) zugeordnet (Ort II oder Ort I):

$$L_k \rightarrow P(X_i; Y_j)$$
$$F_l \rightarrow P(X_i; Y_j)$$
$$\overline{L_k \rightarrow F_l; F_l \rightarrow L_k.}$$

7. Die Zuordnung $L_k \rightarrow F_l$ bzw. $F_l \rightarrow L_k$ wird gespeichert.

Das vorgeschlagene Verfahren ist in seiner Anwendung – wie vorstehend bereits erwähnt – nicht auf Magazine beschränkt, in denen die Probengefäß-Positionen nach Orthogonalkoordinaten festgelegt sind, sondern es sind alle denkbaren Anordnungen verwendbar, bei denen die Probengefäß-Positionen eindeutig definiert werden können. So lassen sich beispielsweise Magazine verwenden, bei denen die Stative spiralförmig, in Form konzentrischer Ringe oder als Gurt ausgebildet sind. Entscheidendes Kriterium für die Eignung ist lediglich, daß die einzelne Probengefäß-Position – in welchem Koordinatensystem auch immer – eindeutig definierbar ist. Gleiche Anforderungen gelten für die Rückmeldefähigkeit dieser Positionen, wobei die Rückmeldung an sich keine notwendige Bedingung für das vorgeschlagene Verfahren, sondern lediglich ein Element zur weiteren Erhöhung der Zuordnungssicherheit im Zuge der Probennahme darstellt.

Ebensowenig wie die Magazinkonfiguration hat die Vorgehensweise bei der Lieferantenerkennung bzw. der Lieferantenvorgabe Einfluß auf die Anwendbarkeit des vorgeschlagenen Verfahrens. Gleichgültig, ob die Kennung des Lieferanten erst vor Ort der Milchannahme gelesen wird oder ob mit Hilfe des vorstehend beschriebenen, in vielen Anwendungsfällen vorteilhaften, weil den notwendigen Aufwand verringernden, Lieferanten-Vorgabeverfahrens eine Milcherfassungstour im Vorwege determiniert ist – das erfindungsgemäße Verfahren ordnet in eindeutiger und unverwechselbarer Weise einen bestimmten Lieferanten (Probemengenlieferanten) einem bestimmten Probengefäß zu.

## Patentansprüche

1. Verfahren zur Probennahme und Zuordnung zwischen Probemengenlieferant und Probengefäß, insbesondere bei der Milchübernahme aus Behältern verschiedener Lieferanten, identisch mit Probemengenlieferanten, in einen Milchsammelwagen, der mit seinem Annahme- und Meßsystem während der Überführung der Gesamtmenge in einen Sammelbehälter eine Probenmenge entnimmt und diese in ein Probengefäß füllt, dadurch gekennzeichnet, daß an einem ersten Ort die Probengefäße zunächst mit einer Kodierung versehen werden, dann die Kodierung dieser und gegebenenfalls bereits zuvor kodierter Probengefäße mindestens einmal gelesen wird und daraufhin die Probengefäße in eine beliebige, aber definierte Position eines Probengefäßmagazins oder dergleichen verbracht werden, sowie die Zuordnung von Probengefäß zu Probengefäß-Position gespeichert wird, und daß nach diesen Schritten an einem weiteren Ort die Probe eines bestimmten Probemengenlieferanten in ein an einer bestimmten Position befindliches Probengefäß überführt wird und die Kodierung des sich an dieser Position befindenden Probengefäßes der Kennung des bestimmten Probemengenlieferanten zugeordnet und diese Zuordnung gespeichert wird.

2. Verfahren nach Anspruch 1, dadurch gekennzeichnet, daß vor, während oder nach der Abfüllung der Probe eines bestimmten Lieferanten in ein Probengefäß die Position dieses Probengefäßes bzw. die Kodierung des dort vorhandenen Probengefäßes der Kennung des Lieferanten zugeordnet und diese Zuordnung gespeichert wird.

3. Verfahren nach Anspruch 1, dadurch gekennzeichnet, daß bei der Abfüllung der Probe eines bestimmten Lieferanten in ein Probengefäß die Position dieses Probengefäßes bzw. dessen Kodierung ermittelt und zusammen mit der Kennung des Lieferanten gespeichert und gegebenenfalls dargestellt und ausgedruckt wird.

4. Verfahren nach einem der Ansprüche 1 bis 3, dadurch gekennzeichnet, daß die Zuordnung zwischen Probemengenlieferant und Probengefäß entweder an Ort und Stelle der Probennahme (II) oder an einem zentralen Ort, z. B. Molkerei oder Zentrallabor, erfolgt.

5. Verfahren nach einem der Ansprüche 1 bis 4, dadurch gekennzeichnet, daß die Probengefäße (10) unverwechselbar in einem Probengefäßmagazin (2) oder dergleichen untergebracht sind.

6. Verfahren nach einem der Ansprüche 1 bis 5, dadurch gekennzeichnet, daß die Probengefäße (10) vor der Kodierung und Unterbringung in dem Probengefäßmagazin (2) aufbereitet, beispielsweise gereinigt, mit Hemmstoff befüllt und verkorkt werden.

## Claims

1. Process for sampling and allocating sample supplier and sample vessel, in particular for the milk transfer from containers of different suppliers, identical with the sample supplier, in a milk collection tank, which by means of its receiving and measuring system draws a sample while the total quantity is transferred into the collection tank and fills said sample into a sample vessel, characterized in that at a first place the sample vessels are provided with a code first, then the codes of said sample vessels and optionally of sample vessels already codified before are read at least once and then said sample vessels are transferred to an arbitrary yet defined position of a sample vessel depot or the like, and that the allocation of sample vessel to sample vessel-position is memorized, and that subsequent to these steps the sample of a specific sample supplier is transferred to a sample vessel located at a specific position and the codifying of the sample vessel located at said position is allocated to the code of the specific sample supplier and said allocation is memorized.

2. Process according to claim 1, characterized in that before, during or after the filling of the sample of a specific supplier into a sample vessel the position of said sample vessel or the code of the sample vessel existing there is allocated to the code of the supplier and said allocation is memorized.

3. Process according to claim 1, characterized in that during the filling of the sample of a specific supplier into a sample vessel the position of said sample vessel and/or its code are determined and memorized together with the code of the supplier and optionally displayed and printed.

4. Process according to any one of claims 1 to 3, characterized in that the allocation between sample supplier and sample vessel is performed either at the place of sampling takes (II) or at a central place, e.g. diary or central laboratory.

5. Process according to any one of claims 1 to 4, characterized in that the sample vessels (10) are stored unmistakably in a sample vessel depot (3) or the like.

6. Process according to any one of claims 1 to 5, characterized in that before the codifying and storing in the sample vessel depot (2) the sample vessels (10) are prepared, e.g. by cleaning, filling with inhibitors and corking.

## Revendications

1. Procédé pour le prélèvement d'échantillons et la corrélation entre le fournisseur de quantités échantillonnées et le récipient d'échantillons, en particulier lors de la prise en charge de lait provenant de fournisseurs différents s'identifiant aux fournisseurs de quantités échantillonnées, dans un véhicule de collecte de lait, qui prélève une quantité échantillon au moyen de son système de réception et de mesure pendant le transfert de la quantité totale dans un récipient collecteur et verse cette quantité échantillon dans un récipient d'échantillon, caractérisé en ce que les récipients d'échantillons sont d'abord pourvus en un premier endroit d'un codage, le codage de ces récipients d'échantillons, le cas échéant déjà codés préalablement, est ensuite au moins lu une fois, après quoi les récipients d'échantillons sont placés dans une position facultative, mais définie, dans un magasin de récipients d'échantillons ou similaire, et la corrélation entre le récipient d'échantillons et sa position est mémorisée, et en ce que, après ces opérations, l'échantillon d'un fournisseur déterminé de quantités échantillonnées est, à un autre endroit, transféré dans un récipient d'échantillons se trouvant dans une position déterminée, le codage du récipient d'échantillons se trouvant dans cette position est affecté à l'identification du fournisseur de quantités échantillonnées et cette affectation est mémorisée.

2. Procédé selon la revendication 1, caractérisé en ce que avant, pendant ou après le déversement de l'échantillon d'un fournisseur déterminé dans un récipient d'échantillons, la position de ce récipient d'échantillons, ou le codage du récipient d'échantillons se trouvant dans cette position, est affectée à l'identification du fournisseur et cette affectation est mémorisée.

3. Procédé selon la revendication 1, caractérisé en ce que lors du déversement de l'échantillon d'un fournisseur déterminé dans un récipient d'échantillons, la position de ce récipient d'échantillons, ou son codage, est déterminée, est mémorisée en même temps que l'identité du fournisseur et, le cas échéant, représentée et imprimée.

4. Procédé selon l'une des revendications 1 à 3, caractérisé en ce que l'affectation du fournisseur de quantités échantillonnées au récipient d'échantillons est effectuée, soit à l'endroit du prélèvement d'échantillons (II), soit en un endroit central tel que laiterie ou laboratoire central.

5. Procédé selon l'une des revendications 1 à 4, caractérisé en ce que les récipients d'échantillons (10) sont placés sans possibilité de confusion dans un magasin de récipients d'échantillons (2) ou similaire.

6. Procédé selon l'une des revendications 1 à 5, caractérisé en ce que les récipients d'échantillons (10) sont préparés avant le codage et la mise en place dans le magasin de récipients d'échantillons (2), par exemple nettoyés, remplis d'un inhibiteur et bouchés.